(19) Europäisches Patentamt
European Patent Office
Office européen des brevets

(11) **EP 3 471 016 B1**

(12) **FASCICULE DE BREVET EUROPEEN**

(45) Date de publication et mention de la délivrance du brevet:
**13.12.2023 Bulletin 2023/50**

(21) Numéro de dépôt: **18199482.3**

(22) Date de dépôt: **10.10.2018**

(51) Classification Internationale des Brevets (IPC):
**G06V 10/143** *(2022.01)* **G06V 40/13** *(2022.01)*

(52) Classification Coopérative des Brevets (CPC):
**G06V 40/1324; G06V 10/143**

(54) **PROCÉDÉ DE DÉTECTION D'UNE PRÉSENCE D'UNE PARTIE CORPORELLE PORTEUSE D'UNE EMPREINTE DIGITALE SUR UN CAPTEUR D EMPREINTES DIGITALES**

PRÄSENZERKENNUNGSVERFAHREN EINES KÖRPERTEILS, DER EINEN FINGERABDRUCK AUF EINEM FINGERABDRUCKSENSOR ERZEUGEN KANN

METHOD FOR DETECTING A PRESENCE OF A BODY PART CARRYING A FINGERPRINT ON A FINGERPRINT SENSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.10.2017 FR 1759524**

(43) Date de publication de la demande:
**17.04.2019 Bulletin 2019/16**

(73) Titulaire: **Idemia Identity & Security France
92400 Courbevoie (FR)**

(72) Inventeur: **DUMONT, Denis
92130 ISSY LES MOULINEAUX (FR)**

(74) Mandataire: **Idemia
2, place Samuel de Champlain
92400 Courbevoie (FR)**

(56) Documents cités:
**EP-A1- 1 810 221 WO-A2-2006/074407
FR-A1- 2 915 008**

**Description**

L'invention concerne un procédé de détection d'une présence d'une partie corporelle porteuse d'une empreinte digitale sur un capteur d'empreintes digitales et d'un dispositif mettant en oeuvre le procédé.

**Contexte de l'invention**

L'utilisation d'empreintes digitales, par exemple du type empreintes d'un doigt, d'une pluralité de doigts, d'une paume de main, permet de sécuriser des accès à des bâtiments ou à des machines. Une telle technologie permet de s'affranchir de codes d'accès ou de cartes qui peuvent être prêtés, volés ou falsifiés. L'utilisation de cette technologie permet de renforcer la sécurité dans la mesure où la probabilité que deux personnes aient deux empreintes digitales identiques est quasiment nulle. Le document FR2915008A1 décrit un tel capteur d'empreintes digitales.

Un dispositif de capture d'une empreinte digitale permet de capturer une image d'une empreinte digitale. Dans le cas d'une identification, cette empreinte est comparée avec un ensemble d'empreintes digitales de référence contenues dans une base de données. Dans le cas d'une authentification, cette empreinte est comparée à une seule empreinte digitale. La comparaison permet de déterminer si l'empreinte digitale capturée appartient ou non à une personne référencée dans la base de données ou si la personne est bien celle qu'elle prétend être.

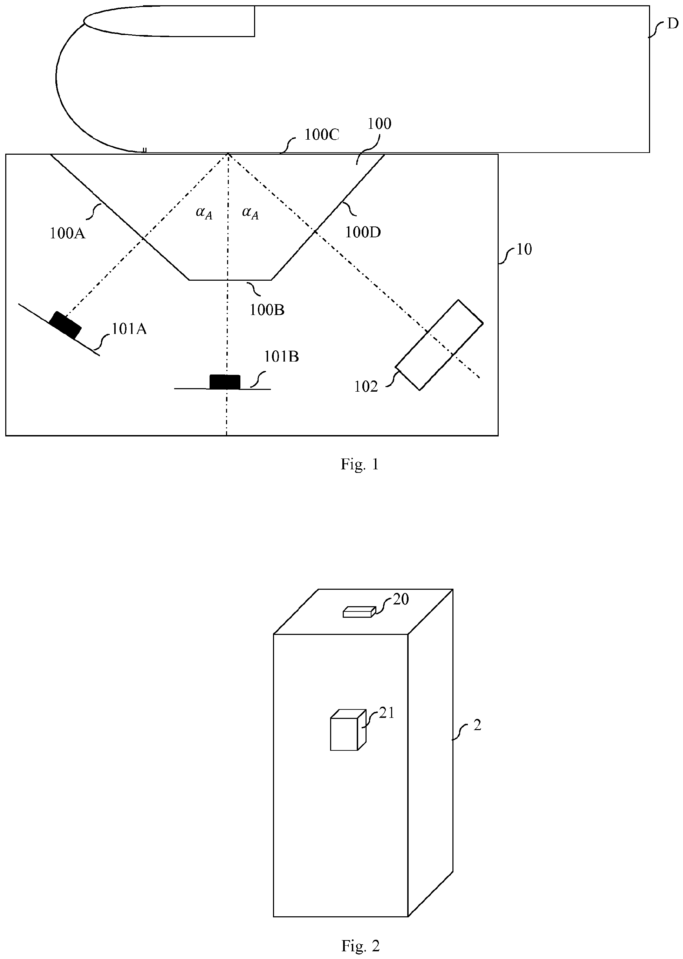

La Fig. 1 décrit schématiquement un dispositif de capture d'une empreinte digitale utilisant le principe de la réflexion totale et pouvant fonctionner en fond clair ou en fond sombre.

Le dispositif 10 décrit en Fig. 1 comprend un prisme 100, deux sources lumineuses 101A et 101B, et un système optique 102 tel que par exemple un capteur d'images CCD (Dispositif à transfert de charges, « Charge-Coupled Device ») ou CMOS (« Complementary Métal Oxide Semiconductor » en terminologie anglo-saxonne) et une ou plusieurs lentilles.

La source lumineuse 101A génère un faisceau lumineux qui traverse une première face 100A du prisme 100 jusqu'à une deuxième face 100C du prisme 100 où est positionnée une partie corporelle porteuse d'une empreinte digitale (ici un doigt D). L'empreinte digitale est constituée de vallées et de crêtes. Le faisceau lumineux généré par la source lumineuse 101A forme un angle incident $\alpha_A$ avec la normale à la face 100C. L'angle $\alpha_A$ est supérieur à un angle critique $\theta_c$ et inférieur à un angle limite $\theta_l$.

La face 100C sépare un premier milieu correspondant au prisme 100 d'un deuxième milieu correspondant à l'air ou au doigt D. L'angle critique $\theta_c$ (resp. l'angle limite $\theta_l$) est défini comme un angle au-delà duquel une réflexion totale se produit lorsqu'un faisceau atteint la face 100C quand le deuxième milieu est l'air (resp. quand le deuxième milieu est le doigt D).

Quand le doigt D est posé sur la face 100C, le faisceau lumineux généré par la source lumineuse 101A subit une réflexion totale lorsque, à la position frappée par le faisceau lumineux sur la face 100C, le doigt forme une vallée, *i.e.* il y a de l'air entre la face 100C et le doigt D. Lorsque, à la position frappée par le faisceau lumineux sur la face 100C, le doigt forme une crête, *i.e.* la peau du doigt est en contact direct avec la face 100C, il n'y a pas réflexion totale. La réflexion totale est alors dite frustrée et le faisceau lumineux est diffusé dans le doigt D.

Après réflexion sur la face 100C, le faisceau lumineux traverse la quatrième face 100D et atteint le système optique 102. Le système optique 102 forme alors une image de l'empreinte digitale à fort contraste entre les vallées et les crêtes. Les vallées correspondent à des faisceaux totalement réfléchis par la face 100C et apparaissent donc très lumineuses dans l'image. Les crêtes correspondent à des faisceaux lumineux diffusés, en partie absorbés dans le doigt D et qui sont ressortis du doigt pour atteindre le système optique 102. Les crêtes apparaissent donc plus sombres dans l'image.

Le système optique 102 reçoit donc à la fois des faisceaux lumineux réfléchis par la face 100C et diffusés dans le doigt D. Le dispositif formé de la source lumineuse 101A, du prisme 100 et du système optique 102 est un dispositif utilisant le principe de réflexion totale à fond clair. On peut trouver un dispositif similaire dans le brevet américain US3200701.

Le dispositif formé de la source lumineuse 101B, du prisme 100 et du système optique 102 fonctionne selon un principe différent. La source lumineuse 101B génère un faisceau lumineux qui traverse une troisième face 100B du prisme 100 jusqu'à la deuxième face 100C où est positionné le doigt D. Le faisceau lumineux généré par la source lumineuse 101B forme un angle incident $\alpha_B$ avec la normale à la face 100C inférieur à l'angle critique $\theta_c$ (ici, l'angle incident $\alpha_B$ est de zéro degré). Il n'y a donc pas de réflexion totale des rayons de la source 101B dans ce cas, cette source étant utilisée pour éclairer le doigt D.

Le système optique 102 reçoit donc le faisceau lumineux généré par la source lumineuse 101B après diffusion par le doigt D. Le système optique 102 est configuré de sorte à recevoir des faisceaux lumineux après diffusion dans le doigt D formant un angle compris entre l'angle critique $\theta_c$ et l'angle limite $\theta_l$ avec la normale à la face 100C. Là encore, le système optique 102 forme une image de l'empreinte digitale à fort contraste entre les vallées et les crêtes. Les crêtes correspondent à des faisceaux lumineux diffusés par le doigt D et qui sont ressortis du doigt au niveau des crêtes en contact avec la face 100C pour atteindre le système optique 102. Aucun faisceau lumineux diffusé dans le doigt D et ressortant du doigt D au niveau des vallées ne peut atteindre le système optique 102 car ils ne peuvent traverser la couche d'air et ensuite se propager dans le prisme 100 tout en formant un angle par rapport à la normale à la face 100B supérieur à l'angle critique $\theta_c$. Les crêtes apparaissent donc plus lumineuses dans l'image d'empreinte que les vallées. Le dispositif formé de la source lumineuse 101B, du prisme 100 et du système optique 102 est un dispositif utilisant le principe de réflexion totale à fond sombre. On peut trouver un dispositif similaire dans le brevet français FR2757974.

L'angle critique $\theta_c$ est donné par la formule suivante :

$$\theta_c = arcsin\left(\frac{n_0}{n_1}\right)$$

$n_1$ étant l'indice de réfraction du prisme et $n_0$ étant l'indice de réfraction de l'air ou du doigt. Pour un indice de réfraction de l'air égal à « 1 » et un indice de réfraction du prisme égal à « 1.5 », on obtient un angle critique $\theta_c$ = 41.8 degrés. L'indice de réfraction de la peau est, dans le domaine du visible, compris entre « 1.41 » et « 1.47 ». En considérant la valeur minimale de « 1.41 », on obtient donc un angle limite $\theta_l$ de « 70 » degrés. En considérant la valeur maximale, on obtient un angle $\theta_l^{max}$ de « 76 » degrés.

Une fonction de détection de présence de parties corporelles est généralement associée à un capteur d'empreintes digitales afin de déclencher un traitement lorsqu'une partie corporelle est détectée comme étant posée sur le capteur d'empreintes digitales. La fonction de détection de présence est sensible car elle ne doit pas déclencher de traitement en présence d'une trace sur le capteur d'empreintes digitales. Cette trace (saletés, sueur, ou autres résidus), aussi appelée *latente,* a pu être laissée par de précédentes applications d'un doigt sur le capteur d'empreintes digitales. Il est connu que les traces peuvent générer une information similaire à certains types de parties corporelles tels que des doigts secs. Il devient alors difficile de discriminer des doigts secs de latentes. Ce problème est notamment rencontré par des capteurs d'empreintes utilisant une technologie d'acquisition optique tels que les capteurs utilisant le principe de réflexion totale à fond sombre ou à fond clair décrits en relation avec la Fig. 1.

Pour discriminer une partie corporelle posée sur un capteur d'empreintes digitales d'une latente, il est connu de réaliser une première image avec une source lumineuse du capteur d'empreintes digitales allumée et une deuxième image avec la source lumineuse éteinte. Ce procédé est lent car il nécessite deux acquisitions d'images et ne fonctionne pas en cas de lumière ambiante forte (par exemple, en plein soleil).

Il est souhaitable de pallier ces inconvénients de l'état de la technique. Il est notamment souhaitable de proposer un procédé et un dispositif qui permettent de mieux distinguer une latente d'un doigt, y compris en présence d'un doigt sec. Par ailleurs, ce procédé doit être simple à mettre en oeuvre.

## EXPOSE DE L'INVENTION

Selon un premier aspect de l'invention, l'invention concerne un procédé de détection d'une présence d'une partie corporelle porteuse d'une empreinte digitale suivant l'une des revendications 1, 3 et 4.

L'invention permet, à partir d'une seule acquisition d'images et en tirant profit des propriétés de réflexion, de diffusion et d'absorption différentes d'une partie corporelle, d'identifier rapidement et simplement une présence d'une partie corporelle sur un capteur d'images. Ainsi, la distinction entre une latente et un doigt sec est plus simple.

Les revendications 2 et 5 concernent des modes de réalisation du procédé.

Selon un deuxième aspect de l'invention, l'invention concerne un dispositif permettant de détecter une présence d'une partie corporelle porteuse d'une empreinte digitale selon les revendications 6, 8 et 9.

Les revendications 7 et 10 concernent des modes de réalisation du dispositif.

Selon un troisième aspect de l'invention, l'invention concerne un capteur d'empreintes digitales selon la revendication 11.

Selon un quatrième aspect de l'invention, l'invention concerne un programme d'ordinateur selon la revendication 12.

Selon un cinquième aspect de l'invention, l'invention concerne des moyens de stockage selon la revendication 13.

## BREVE DESCRIPTION DES DESSINS

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :

- la Fig. 1 décrit schématiquement un dispositif de capture d'une empreinte digitale de l'art antérieur travaillant en réflexion totale et pouvant fonctionner en fond clair ou en fond sombre ;
- la Fig. 2 illustre schématiquement un équipement comprenant un dispositif de capture d'une empreinte digitale selon l'invention ;
- la Fig. 3 illustre schématiquement un exemple d'architecture matérielle d'un module de traitement mettant en oeuvre le procédé de détection d'une présence d'une partie corporelle porteuse d'une empreinte digitale ;
- la Fig. 4 illustre schématiquement un exemple de procédé de détection d'une présence d'une partie corporelle porteuse d'une empreinte digitale ;
- la Fig. 5A illustre schématiquement un détail d'une première mise en oeuvre particulière du procédé de détection d'une présence d'une partie corporelle porteuse d'une empreinte digitale ;

- la Fig. 5B illustre schématiquement un détail d'une deuxième mise en oeuvre particulière du procédé de détection d'une présence d'une partie corporelle porteuse d'une empreinte digitale ; et,
- la Fig. 5C illustre schématiquement un détail d'une troisième mise en oeuvre particulière du procédé de détection d'une présence d'une partie corporelle porteuse d'une empreinte digitale.

## DESCRIPTION DETAILLEE DE DIVERS MODES DE REALISATION

La description qui suit détaille plus particulièrement des modes de réalisation de la présente invention dans un contexte d'un capteur d'empreintes digitales utilisé dans un équipement de contrôle d'accès à une salle ou à un bâtiment. L'invention peut s'appliquer à d'autres équipements pouvant comprendre un dispositif de capture d'une empreinte d'une partie corporelle tel qu'un ordinateur, une tablette, un téléphone intelligent, etc. Par ailleurs, l'invention est décrite dans un contexte où la partie corporelle est un doigt. Elle s'applique toutefois à d'autres parties corporelles telles que plusieurs doigts, une paume de main, etc. De plus, nous décrivons l'invention dans un contexte où le dispositif de capture d'empreintes digitales est celui décrit en relation avec la Fig. 1. D'autres types de capteurs d'empreintes peuvent être utilisés et notamment des capteurs d'empreintes dans lesquels le prisme 100 est remplacé par une lame transparente.

La **Fig. 2** illustre schématiquement un équipement comprenant un dispositif de capture d'une empreinte digitale selon l'invention.

L'équipement 2 est un équipement de contrôle d'accès prenant une forme de borne rectangulaire comprenant sur une face supérieure un dispositif de capture d'empreintes digitales 20. Lorsqu'un utilisateur souhaite accéder à une salle ou à un bâtiment, il pose un doigt sur le capteur d'empreintes digitales 20. Le capteur d'empreintes digitales 20 déclenche une ouverture d'une porte lorsque l'utilisateur est reconnu. Le capteur d'empreintes digitales 20 est par exemple celui décrit en relation avec la Fig. 1. Nous supposons par la suite que chaque source lumineuse 101A et 101B produit une lumière ayant une puissance uniforme dans toutes les longueurs d'onde captées par le système optique 102. De même, on considère par la suite que le système optique 102 a une sensibilité identique pour chaque longueur d'onde reçue. Si ce n'était pas le cas, il serait préférable de rééquilibrer chaque composante produite par le système optique 102 afin que celles-ci soient comparables entre elles. Comme nous le décrivons par la suite, dans certains modes de réalisation, ce capteur d'empreintes digitales 20 utilise le principe de réflexion totale à fond clair et dans d'autres celui de réflexion totale à fond sombre. L'équipement 2 comprend un module de traitement 21 apte à mettre en oeuvre le procédé selon l'invention.

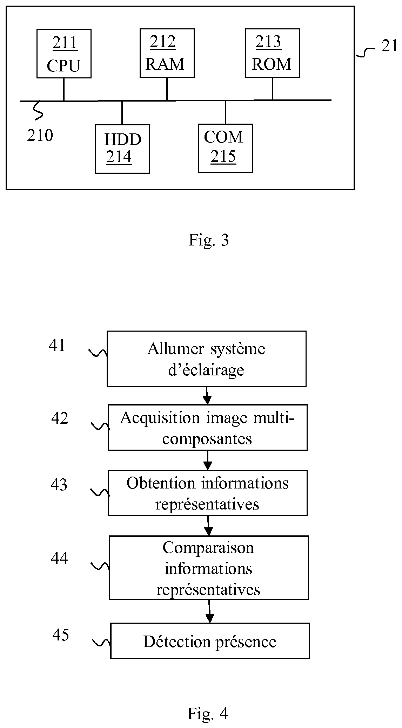

La **Fig. 3** illustre schématiquement un exemple d'architecture matérielle du module de traitement 21.

Selon l'exemple d'architecture matérielle représenté à la Fig. 3, le module de traitement 21 comprend alors, reliés par un bus de communication 210 : un processeur ou CPU (« Central Processing Unit » en anglais) 211 ; une mémoire vive RAM (« Random Access Memory » en anglais) 212 ; une mémoire morte ROM (« Read Only Memory » en anglais) 213 ; une unité de stockage telle qu'un disque dur ou un lecteur de support de stockage, tel qu'un lecteur de cartes SD (« Secure Digital » en anglais) 214 ; au moins une interface de communication 215 permettant au module de traitement 21 de communiquer avec le capteur d'empreintes digitales 20 et de le contrôler.

Le processeur 211 est capable d'exécuter des instructions chargées dans la RAM 212 à partir de la ROM 213, d'une mémoire externe (non représentée), d'un support de stockage (tel qu'une carte SD), ou d'un réseau de communication. Lorsque le module d'analyse 21 est mis sous tension, le processeur 211 est capable de lire de la RAM 212 des instructions et de les exécuter. Ces instructions forment un programme d'ordinateur causant la mise en oeuvre, par le processeur 211, du procédé décrit en relation avec la Fig. 4.

Le procédé décrit en relation avec la Fig. 4 peut être implémenté sous forme logicielle par exécution d'un ensemble d'instructions par une machine programmable, par exemple un DSP (« Digital Signal Processor » en anglais),un microcontrôleur ou un GPU (processeur graphique, « Graphics Processing Unit » en terminologie anglo-saxonne), ou être implémenté sous forme matérielle par une machine ou un composant dédié, par exemple un FPGA (« Field-Programmable Gate Array » en anglais) ou un ASIC (« Application-Specific Integrated Circuit » en anglais).

On note que le module de traitement 21 aurait tout aussi bien pu être compris dans le capteur d'empreintes digitales 20.

La **Fig. 4** illustre schématiquement un exemple de procédé de détection d'une présence d'une partie corporelle porteuse d'une empreinte digitale.

Dans une étape 41, le module de traitement 21 déclenche un allumage d'au moins une des sources lumineuses 101A ou 101B du capteur d'empreintes digitales 20. Dans un mode de réalisation, lorsqu'un utilisateur pose un doigt sur le capteur d'empreintes digitales 20, il appuie sur un bouton pour déclencher l'allumage.

Dans une étape 42, le module de traitement 21 déclenche une acquisition d'une image multi-composantes par le capteur d'images 102. Dans un mode de réalisation, le capteur d'images 102 comprend une matrice de pluralités de photorécepteurs, chaque pluralité comprenant au moins trois photorécepteurs captant des gammes de longueurs d'onde prédéterminées différentes. Chaque pixel d'une image générée par le capteur d'images 102 comprenant une pluralité de composantes, chaque composante est fournie par au moins un des photorécepteurs de la pluralité associée à ce pixel. Dans un mode de réalisation, chaque pluralité comprend quatre photorécepteurs, deux photorécepteurs captent des rayons lumineux dans une gamme de longueurs d'onde correspondant au vert et fournissant une composante verte, un photorécepteur capte des rayons lumineux dans une gamme de longueurs d'onde correspondant au bleu et fournissant une composante bleue, un photorécepteur capte des rayons lumineux dans une gamme de longueurs d'onde correspondant au rouge et fournissant une composante rouge.

Dans une étape 43, le module de traitement 21, obtient, pour chaque composante de couleur de l'image multi-composantes, une information représentative d'un signal généré par ledit capteur d'images pour ladite composante de couleur.

Dans une étape 44, le module de traitement 21 compare lesdites informations représentatives entre elles et détecte, dans une étape 45, une présence d'une partie corporelle en fonction d'un résultat de la comparaison.

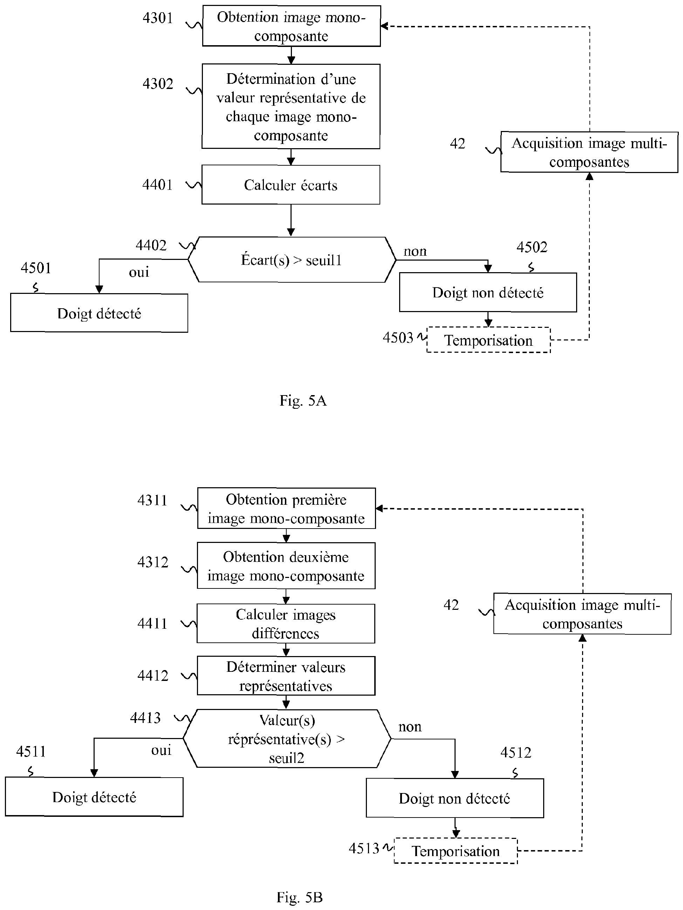

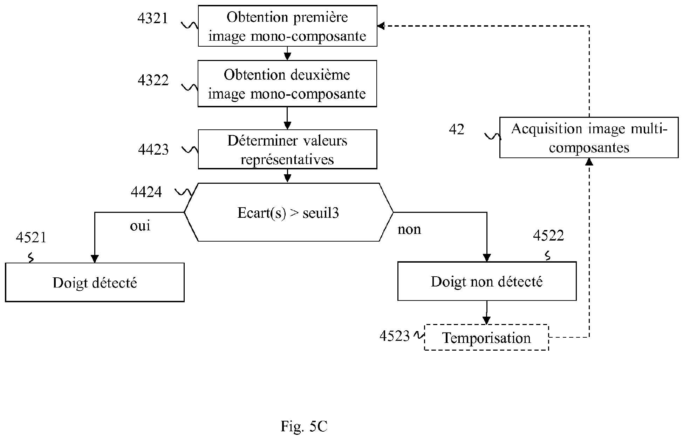

Nous décrivons par la suite en relation avec les Figs. 5A, 5B et 5C des modes de réalisation détaillés des étapes 43, 44 et 45.

La **Fig. 5A** illustre schématiquement un détail d'une première mise en oeuvre particulière du procédé de détection d'une présence d'une partie corporelle porteuse d'une empreinte digitale.

Dans la mise en oeuvre décrite en relation avec la Fig. 5A, le capteur d'empreintes digitales 20 fonctionne selon le principe de réflexion totale à fond sombre et la source lumineuse génère une lumière dont les longueurs d'onde sont comprises exclusivement dans une des gammes de longueurs d'onde prédéfinies, dite gamme de longueurs d'onde d'éclairage.

Dans une étape 4301, le module de traitement 21 forme une image mono-composante à partir de chaque composante de l'image multi-composantes. Si par exemple, chaque pixel de l'image multi-composantes comprend une composante rouge, une composante verte et une composante bleue, le module de traitement forme une image mono-composante comprenant la composante rouge, une image mono-composante comprenant la composante verte et une image mono-composante comprenant la composante bleue.

Dans une étape 4302, le module de traitement 21 détermine une valeur représentative de chaque image mono-composante. Dans un mode de réalisation, pour chaque image mono-composante, le module de traitement 21 calcule une valeur de dynamique en chaque pixel de l'image. Pour ce faire, pour chaque pixel d'une image mono-composante, le module de traitement 21 définit un voisinage autour dudit pixel, par exemple sous forme d'une matrice carrée de côté prédéfini centré sur ledit pixel et calcule une valeur de dynamique locale sous forme d'une moyenne des valeurs des pixels compris dans ce voisinage. Dans un mode de réalisation, le côté de la matrice carrée est de dix pixels. Une fois tous les pixels de l'image mono-composante parcourus, le module de traitement calcule une moyenne des valeurs des dynamiques locales de toute l'image pour obtenir une valeur de dynamique globale de l'image mono-composante. La valeur de dynamique globale d'une image mono-composante forme une valeur représentative de ladite image mono-composante.

Les étapes 4301 et 4302 détaillent l'étape 43.

Dans une étape 4401, le module de traitement 21 calcule un écart entre les valeurs représentatives de chaque image mono-composante prises deux à deux. Lorsque l'image multi-composantes comprend trois composantes (Rouge, Vert, Bleu), le module de traitement 21 calcule trois écarts.

Il est connu qu'un doigt posé sur le capteur d'empreintes digitales 20 va générer une image essentiellement dans la composante correspondant à la gamme de longueurs d'onde émise par la source lumineuse alors qu'une latente éclairée par une lumière ambiante génère une image dans chaque composante. Ainsi, si la valeur représentative d'une composante correspondant à la gamme de longueurs d'onde émise par la source lumineuse est très différente des autres valeurs représentatives, on peut en déduire qu'un doigt est posé sur le capteur d'empreintes digitales 20. Par contre, si toutes les valeurs représentatives sont approximativement égales, on peut en déduire qu'aucun doigt n'est posé sur le capteur d'empreintes digitales.

Dans une étape 4402, chaque écart calculé est comparé à un premier seuil prédéterminé. Le premier seuil prédéterminé représente un écart élevé au-delà duquel la probabilité pour que le capteur d'empreintes digitales 20 soit en présence d'un doigt est très élevée et a par exemple été déterminé en activant le capteur d'empreintes digitales 20 de la mise en oeuvre de la Fig. 5A sur un large panel de doigts et sur des latentes laissées sur le capteur d'empreintes digitales 20 par lesdits doigts.

Les étapes 4401 et 4402 sont un détail de l'étape 44.

Dans une étape 4501, lorsque l'écart entre la valeur représentative déterminée pour la composante de couleur correspondant à la gamme de longueurs d'onde d'éclairage et les autres valeurs représentatives est supérieur au premier seuil prédéterminé, le module de traitement 21 détermine que le capteur d'empreintes digitales 20 est en présence d'un doigt.

Sinon, le module de traitement 21 détermine lors d'une étape 4502 que le capteur d'empreintes digitales 20 n'est pas en présence d'un doigt.

Lorsque l'écart entre la valeur représentative déterminée pour la composante de couleur correspondant à la gamme de longueurs d'onde d'éclairage et les autres valeurs représentatives est supérieur au premier seuil prédéterminé, la probabilité pour que le capteur d'empreintes digitales 20 soit en présence d'un doigt est très élevée. Par contre, lorsque l'écart entre la valeur représentative déterminée pour la composante de couleur correspondant à la gamme de longueurs d'onde d'éclairage et les autres valeurs représentatives est inférieur au premier seuil prédéterminé, on ne peut pas conclure avec certitude que le capteur d'empreintes digitales 20 n'est pas en présence d'un doigt. En effet, les caractéristiques de l'image multi-composantes acquise lors de l'étape 42 dépendent de l'état du doigt au moment de cette acquisition et peuvent évoluer dans le temps. Ainsi, un doigt sec lors de l'acquisition de l'image multi-composantes peut, peu à peu, devenir humide et ainsi permettre une acquisition d'une image multi-composantes plus exploitable.

Dans un mode de réalisation, suite à l'étape 4502, le module de traitement 21 met en oeuvre une étape optionnelle 4503 de temporisation. Lors de cette étape, le module de traitement 21 se met en attente d'une durée prédéfinie de quelques millisecondes (par exemple 10 millisecondes), remet en oeuvre l'étape 42 puis revient à l'étape 4301 de manière à remettre en oeuvre l'algorithme de la Fig. 5A. Le module de traitement 21 peut boucler ainsi sur l'algorithme de la Fig. 5A un nombre de fois prédéfini $N$ ($N$ est par exemple égal à 5). Si au cours d'une des mises en oeuvre de l'étape 4402, l'écart entre la valeur représentative déterminée pour la composante de couleur correspondant à la gamme de longueurs d'onde d'éclairage et les autres valeurs représentatives est supérieur au premier seuil prédéterminé, le module de traitement 21 détermine que le capteur d'empreintes digitales 20 est en présence d'un doigt et l'exécution du procédé décrit en relation avec la Fig. 5A s'arrête. Si après N boucles, aucun doigt n'est détecté, le module de traitement 21 considère définitivement qu'il n'y a pas de doigts sur le capteur d'empreintes digitales 20.

Les étapes 4501, 4502 et 4503 sont un détail de l'étape 45.

La **Fig. 5B** illustre schématiquement un détail d'une deuxième mise en oeuvre particulière du procédé de détection d'une présence d'une partie corporelle porteuse d'une empreinte digitale.

Dans la mise en oeuvre décrite en relation avec la Fig. 5B, le capteur d'empreintes digitales 20 fonctionne selon le principe de réflexion totale à fond sombre et la source lumineuse produit une lumière dont les longueurs d'onde sont situées dans une première gamme de longueurs d'onde prédéfinie et dans au moins une deuxième gamme de longueurs d'onde prédéfinie.

Dans une étape 4311, le module de traitement 21 obtient à partir de l'image multi-composantes, une première image mono-composante comprenant la composante de l'image multi-composantes correspondant à la première gamme de longueurs d'onde prédéfinie.

Dans une étape 4312, le module de traitement 21 obtient à partir de l'image multi-composantes, pour chaque deuxième gamme de longueurs d'onde prédéfinie, une deuxième image mono-composante comprenant la composante de l'image multi-composantes correspondant à ladite deuxième gamme de longueur d'onde prédéfinie.

Dans ce mode de réalisation, la première image mono-composante et chaque deuxième image mono-composante constituent l'information représentative d'un signal généré par ledit capteur d'images pour chaque composante.

Les étapes 4311 et 4312 sont un détail de l'étape 43.

Dans une étape 4411, le module de traitement 21 calcule une différence entre chaque image mono-composante prise deux à deux. Si le module de traitement 21 a obtenu trois images mono-composantes, il obtient alors trois images de différence.

Lorsqu'un doigt est posé sur le capteur d'empreintes digitales 20, la réponse spectrale dudit doigt implique qu'au moins une des différences entre deux composantes est significative. La réponse spectrale d'une latente est différente puisque, dans ce cas, chaque différence est faible.

Dans une étape 4412, le module de traitement 21 calcule une valeur de dynamique globale tel que décrit en relation avec l'étape 4302 pour chaque image mono-composante.

Dans une étape 4413 chaque valeur de dynamique globale est comparée à un deuxième seuil prédéterminé. Le deuxième seuil prédéterminé représente un écart significatif au-delà duquel la probabilité pour que le capteur d'empreintes digitales 20 soit en présence d'un doigt est très élevée et a par exemple été déterminé en activant le capteur d'empreintes digitales 20 de la mise en oeuvre de la Fig. 5B sur un large panel de doigts et sur des latentes laissées sur le capteur d'empreintes digitales 20 par lesdits doigts.

Les étapes 4411, 4412 et 4413 sont un détail de l'étape 44.

Lorsqu'au moins un écart est supérieur au deuxième seuil, le module de traitement 21 considère lors d'une étape 4511 que le capteur d'empreintes digitales 20 est en présence d'un doigt. Sinon, le module de traitement 21 considère lors d'une étape 4512 que le capteur d'empreintes digitales 20 n'est pas en présence d'un doigt.

Les étapes 4511 et 4512 correspondent à un détail de l'étape 45.

Dans un mode de réalisation, suite à l'étape 4512, le module de traitement 21 met en oeuvre une étape 4513 optionnelle identique à l'étape 4503 et boucle sur l'algorithme de la Fig. 5B un nombre de fois prédéfini tel que décrit en relation avec la Fig. 5A.

Dans un mode de réalisation, la première gamme de longueurs d'onde prédéfinie correspond au rouge et une deuxième gamme de longueurs d'onde prédéfinie correspond au vert ou au bleu.

La **Fig. 5C** illustre schématiquement un détail d'une troisième mise en oeuvre particulière du procédé de détection d'une présence d'une partie corporelle porteuse d'une empreinte digitale.

Dans la mise en oeuvre décrite en relation avec la Fig. 5C, le capteur d'empreintes digitales 20 fonctionne selon le principe de réflexion totale à fond clair et la source lumineuse produit une lumière dont les longueurs d'onde sont situées dans une première gamme de longueurs d'onde prédéfinie et dans au moins une deuxième gamme de longueurs d'onde prédéfinie. On suppose que la première gamme de longueurs d'onde prédéfinie correspond au rouge et qu'une deuxième gamme de longueurs d'onde prédéfinie correspond au vert ou au bleu. Il est connu qu'un doigt absorbe la lumière différemment en fonction de sa longueur d'onde. Ainsi l'absorption est plus forte pour la lumière bleue et pour la lumière verte que pour la lumière rouge. Par conséquent, un capteur d'images d'un capteur d'empreintes digitales reçoit moins de rayons lumineux correspondant aux longueurs d'onde du vert et du bleu que de rayons lumineux correspondant au rouge lorsqu'un doigt est posé sur le capteur. Par contre, l'absorption par une latente est la même quelle que soit la longueur d'onde. Si une image mono-composante correspondant au vert (ou au bleu) comprend moins d'informations qu'une image mono-composante correspondant au rouge, on peut en déduire que le capteur d'empreintes digitales 20 est en présence d'un doigt. Si l'information contenue dans chaque image mono-composante est identique, on peut en déduire que le capteur d'empreintes digitales 20 n'est pas en présence d'un doigt.

La mise en oeuvre de la Fig. 5C débute par des étapes 4321 et 4322 identiques aux étapes 4311 et 4312.

Dans une étape 4423, le module de traitement 21 calcule une information représentative de chaque image mono-composante. Dans un mode de réalisation, le module de traitement 21 calcule une valeur de dynamique globale telle que décrit en relation avec l'étape 4302 pour chaque image mono-composante.

Dans une étape 4424, le module de traitement 21 compare l'information représentative de l'image mono-composante correspondant au rouge à l'information représentative de l'image mono-composante correspondant au vert et/ou à l'information représentative de l'image mono-composante correspondant au bleu.

Si un écart entre l'information représentative de l'image mono-composante correspondant au rouge et l'information représentative de l'image mono-composante correspondant au vert et/ou au bleu est supérieur à un troisième seuil, le module de traitement 21 considère que le capteur d'empreintes digitales 20 est en présence d'un doigt dans une étape 4521. Sinon, le module de traitement 21 considère que le capteur d'empreintes digitales 20 n'est pas en présence d'un doigt dans une étape 4522.

Le troisième seuil prédéterminé représente un écart significatif au-delà duquel la probabilité pour que le capteur d'empreintes digitales 20 soit en présence d'un doigt est très élevée et a par exemple été déterminé en activant le capteur d'empreintes digitales 20 de la mise en oeuvre de la Fig. 5C sur un large panel de doigts et sur des latentes laissées sur le capteur d'empreintes digitales 20 par lesdits doigts.

Les étapes 4423 et 4424 sont des détails de l'étape 44.

Les étapes 4521 et 4522 sont des détails de l'étape 45.

Dans un mode réalisation, suite à l'étape 4522, le module de traitement 21 met en oeuvre une étape optionnelle 4523 identique à l'étape 4503 et boucle sur l'algorithme décrit en relation avec la Fig. 5C à l'image de ce qui est fait dans l'algorithme de la Fig. 5A.

Dans un mode de réalisation, le capteur d'images 102 fournit successivement des images mono-composantes correspondant chacune à une gamme de longueurs d'onde prédéfinie, les images mono-composantes formant l'image multi-composantes.

**Revendications**

1. Procédé de détection d'une présence d'une partie corporelle porteuse d'une empreinte digitale (D) sur un capteur d'empreintes digitales (20) comprenant un face transparente (100C) sur laquelle doit être posée une partie corporelle (D), un capteur d'images (102), situé en regard de la face transparente (100C), apte à acquérir une image d'une empreinte digitale, une source lumineuse (101A, 101B) permettant d'éclairer une partie corporelle posée sur la face transparente (100C), le procédé comprenant:

allumer (41) la source lumineuse ;
obtenir (42) une image multi-composantes du capteur d'images, chaque composante de couleur de l'image multi-composantes correspondant à une gamme de longueurs d'onde prédéfinie, le capteur d'empreintes digitales fonctionnant selon un principe de réflexion totale à fond sombre, la source lumineuse produisant une lumière dont les longueurs d'onde sont comprises exclusivement dans une des gammes de longueurs d'onde prédéfinies, dite gamme de longueurs d'onde d'éclairage ;
former (4301) une image mono-composante à partir de chaque composante de l'image multi-composantes,
déterminer (4302) une valeur représentative de chaque image mono-composante comprenant de :

calculer, pour chaque pixel de l'image mono-composante, une valeur dynamique locale sous forme d'une moyenne des valeurs des pixels compris dans un voisinage autour dudit pixel,
calculer une moyenne des valeurs de dynamique locale de toute l'image mono-composante pour obtenir une valeur de dynamique globale de l'image mono-composante, la valeur de dynamique globale de l'image mono-composante formant ladite valeur représentative de chaque image mono-composante;

comparer (4401, 4402) les valeurs représentatives de chaque composante entre elles, détecter une présence (4501) d'une partie corporelle lorsqu'un écart entre la valeur représentative déterminée pour la composante de couleur correspondant à la gamme de longueurs d'onde d'éclairage et les autres valeurs représentatives est supérieur à un premier seuil prédéterminé.

**2.** Procédé selon la revendication 1, **caractérisé en ce que** chaque pixel de l'image multi-composantes comprend une composante rouge, une composante verte et une composante bleue, un module de traitement formant une image mono-composante comprenant la composante rouge, une image mono-composante comprenant la composante verte et une image mono-composante comprenant la composante bleue.

**3.** Procédé de détection d'une présence d'une partie corporelle porteuse d'une empreinte digitale (D) sur un capteur d'empreintes digitales (20) comprenant un face transparente (100C) sur laquelle doit être posée une partie corporelle (D), un capteur d'images (102), situé en regard de la face transparente (100C), apte à acquérir une image d'une empreinte digitale, une source lumineuse (101A, 101B) permettant d'éclairer une partie corporelle posée sur la face transparente (100C), le procédé comprenant:

allumer (41) la source lumineuse ;
obtenir (42) une image multi-composantes du capteur d'images, chaque composante de couleur de l'image multi-composantes correspondant à une gamme de longueurs d'onde prédéfinie, le capteur d'empreintes digitales fonctionnant selon un principe de réflexion totale à fond sombre, la source lumineuse produisant une lumière dont les longueurs d'onde sont situées dans une première gamme de longueurs d'onde prédéfinie et dans au moins une deuxième gamme de longueurs d'onde prédéfinie,
obtenir (4311) du capteur d'images une première image mono-composante comprenant la composante de l'image multi-composantes correspondant à la première gamme de longueurs d'onde prédéfinie et (4312) pour chaque deuxième gamme de longueurs d'onde prédéfinie, une deuxième image mono-composante comprenant la composante de l'image multi-composantes correspondant à ladite deuxième gamme de longueurs d'onde prédéfinie, chaque image mono-composante étant une information représentative d'un signal généré par ledit capteur d'images pour une composante de couleur ;
calculer (4411) une image de différence entre la première image et chaque deuxième image et entre chaque deuxième image ;
déterminer (4412) une valeur représentative de chaque image de différence, comprenant de :

calculer, pour chaque pixel de l'image de différence, une valeur dynamique locale sous forme d'une moyenne des valeurs des pixels compris dans un voisinage autour dudit pixel,
calculer une moyenne des valeurs de dynamique locale de toute l'image de différence pour obtenir une valeur de dynamique globale de l'image de différence, la valeur de dynamique globale de l'image de différence formant ladite valeur représentative de chaque image de différence ;

détecter (4511) une présence d'une partie corporelle lorsqu'au moins une des valeurs représentatives est supérieure à un premier seuil prédéterminé.

**4.** Procédé de détection d'une présence d'une partie corporelle porteuse d'une empreinte digitale (D) sur un capteur d'empreintes digitales (20) comprenant un face transparente (100C) sur laquelle doit être posée une partie corporelle (D), un capteur d'images (102), situé en regard de la face transparente (100C), apte à acquérir une image d'une empreinte digitale, une source lumineuse (101A, 101B) permettant d'éclairer une partie corporelle posée sur la face transparente (100C), le procédé comprenant:

allumer (41) la source lumineuse ;
obtenir (42) une image multi-composantes du capteur d'images, chaque composante de couleur de l'image multi-composantes correspondant à une gamme de longueurs d'onde prédéfinie, le capteur d'empreintes digitales fonctionnant selon un principe de réflexion totale à fond clair, la source lumineuse produisant une lumière dont les longueurs d'onde sont situées dans une première gamme de longueurs d'onde prédéfinie et dans au moins une deuxième gamme de longueurs d'onde prédéfinie,
obtenir du capteur d'images une première image mono-composante (4321) comprenant la composante de l'image multi-composantes correspondant à la première gamme de longueurs d'onde prédéfinie et pour chaque deuxième gamme de longueurs d'onde prédéfinie, une deuxième image mono-composante (4322) comprenant la composante de l'image multi-composantes correspondant à ladite deuxième gamme de longueurs d'onde prédéfinie ;

calculer (4423) une valeur représentative de chaque image mono-composante, comprenant de :

calculer, pour chaque pixel de l'image mono-composante, une valeur dynamique locale sous forme d'une moyenne des valeurs des pixels compris dans un voisinage autour dudit pixel,

calculer une moyenne des valeurs de dynamique locale de toute l'image mono-composante pour obtenir une valeur de dynamique globale de l'image mono-composante, la valeur de dynamique globale de l'image mono-composante formant ladite valeur représentative de chaque image mono-composante ; comparer (4424) les valeurs représentatives associées à chaque composante entre elles,

détecter (4521) une présence d'une partie corporelle lorsqu'un écart entre la valeur représentative associée à la première image mono-composante et chaque valeur représentative associée à la deuxième image mono-composante est supérieur à un premier seuil prédéterminé.

5. Procédé selon la revendication 3 ou 4 **caractérisé en ce que** la première composante est une composante rouge et chaque deuxième composante est une composante verte ou bleue.

6. Dispositif permettant de détecter une présence d'une partie corporelle porteuse d'une empreinte digitale (D) sur un capteur d'empreintes digitales (20) comprenant un face transparente (100C) sur laquelle doit être posée une partie corporelle (D), un capteur d'images (102), situé en regard de la face transparente (100C), apte à acquérir une image d'une empreinte digitale, une source lumineuse (101A, 101B) permettant d'éclairer une partie corporelle posée sur la face transparente (100C), le dispositif comprenant:

des moyens d'allumage (41) pour allumer la source lumineuse ;

des moyens d'obtention (42) pour obtenir une image multi-composantes du capteur d'images, chaque composante de l'image multi-composantes correspondant à une gamme de longueurs d'onde prédéfinie, le capteur d'empreintes digitales étant apte à fonctionner selon un principe de réflexion totale à fond sombre, la source lumineuse étant apte à produire une lumière dont les longueurs d'onde sont comprises exclusivement dans une des gammes de longueurs d'onde prédéfinies, dite gamme de longueurs d'onde d'éclairage;

des moyens de formation pour former (4301) une image mono-composante à partir de chaque composante de l'image multi-composantes,

des moyens de détermination pour déterminer (4302) une valeur représentative de chaque image mono-composante, aptes à :

calculer, pour chaque pixel de l'image mono-composante, une valeur dynamique locale sous forme d'une moyenne des valeurs des pixels compris dans un voisinage autour dudit pixel,

calculer une moyenne des valeurs de dynamique locale de toute l'image mono-composante pour obtenir une valeur de dynamique globale de l'image mono-composante, la valeur de dynamique globale de l'image mono-composante formant ladite valeur représentative de chaque image mono-composante;

des moyens de comparaison (4401, 4402) pour comparer les valeurs représentatives de chaque composante entre elles,

des moyens de détection pour détecter une présence (4501) d'une partie corporelle lorsqu'un écart entre la valeur représentative déterminée pour la composante de couleur correspondant à la gamme de longueurs d'onde d'éclairage et les autres valeurs représentatives est supérieur à un premier seuil prédéterminé.

7. Dispositif selon la revendication 6, **caractérisé en ce que** chaque pixel de l'image multi-composantes comprend une composante rouge, une composante verte et une composante bleue, le dispositif comportant un module de traitement apte à former une image mono-composante comprenant la composante rouge, une image mono-composante comprenant la composante verte et une image mono-composante comprenant la composante bleue.

8. Dispositif permettant de détecter une présence d'une partie corporelle porteuse d'une empreinte digitale (D) sur un capteur d'empreintes digitales (20) comprenant un face transparente (100C) sur laquelle doit être posée une partie corporelle (D), un capteur d'images (102), situé en regard de la face transparente (100C), apte à acquérir une image d'une empreinte digitale, une source lumineuse (101A, 101B) permettant d'éclairer une partie corporelle posée sur la face transparente (100C), le dispositif comprenant:

des moyens d'allumage (41) pour allumer la source lumineuse ;

des moyens d'obtention (42) pour obtenir une image multi-composantes du capteur d'images, chaque composante de l'image multi-composantes correspondant à une gamme de longueurs d'onde prédéfinie, le capteur d'empreintes digitales étant apte à fonctionner selon un principe de réflexion totale à fond sombre, la source lumineuse étant apte à produire une lumière dont les longueurs d'onde sont situées dans une première gamme de longueurs d'onde prédéfinie et dans au moins une deuxième gamme de longueurs d'onde prédéfinie,

des moyens d'obtention pour obtenir du capteur d'images une première image mono-composante (4321) comprenant la composante de l'image multi-composantes correspondant à la première gamme de longueurs d'onde prédéfinie et pour chaque deuxième gamme de longueurs d'onde prédéfinie, une deuxième image mono-composante (4322) comprenant la composante de l'image multi-composantes correspondant à ladite deuxième gamme de longueurs d'onde prédéfinie ;

des moyens de calcul pour calculer (4423) une valeur représentative de chaque image mono-composante, aptes à :

> calculer, pour chaque pixel de l'image mono-composante, une valeur dynamique locale sous forme d'une moyenne des valeurs des pixels compris dans un voisinage autour dudit pixel,
> calculer une moyenne des valeurs de dynamique locale de toute l'image mono-composante pour obtenir une valeur de dynamique globale de l'image mono-composante, la valeur de dynamique globale de l'image mono-composante formant ladite valeur représentative de chaque image mono-composante ;

des moyens de comparaison pour comparer (4424) les valeurs représentatives associées à chaque composante entre elles,
des moyens de détection pour détecter (4521) une présence d'une partie corporelle lorsqu'un écart entre la valeur représentative associée à la première image mono-composante et chaque valeur représentative associée à la deuxième image mono-composante est supérieur à un premier seuil prédéterminé.

9. Dispositif permettant de détecter une présence d'une partie corporelle porteuse d'une empreinte digitale (D) sur un capteur d'empreintes digitales (20) comprenant un face transparente (100C) sur laquelle doit être posée une partie corporelle (D), un capteur d'images (102), situé en regard de la face transparente (100C), apte à acquérir une image d'une empreinte digitale, une source lumineuse (101A, 101B) permettant d'éclairer une partie corporelle posée sur la face transparente (100C), le dispositif comprenant:

des moyens d'allumage (41) pour allumer la source lumineuse ;
des moyens d'obtention (42) pour obtenir une image multi-composantes du capteur d'images, chaque composante de l'image multi-composantes correspondant à une gamme de longueurs d'onde prédéfinie, le capteur d'empreintes digitales étant apte à fonctionner selon un principe de réflexion totale à fond clair, la source lumineuse étant apte à produire une lumière dont les longueurs d'onde sont situées dans une première gamme de longueurs d'onde prédéfinie et dans au moins une deuxième gamme de longueurs d'onde prédéfinie,
des moyens d'obtention pour obtenir du capteur d'images une première image mono-composante (4321) comprenant la composante de l'image multi-composantes correspondant à la première gamme de longueurs d'onde prédéfinie et pour chaque deuxième gamme de longueurs d'onde prédéfinie, une deuxième image mono-composante (4322) comprenant la composante de l'image multi-composantes correspondant à ladite deuxième gamme de longueurs d'onde prédéfinie ;
des moyens de calcul pour calculer (4423) une valeur représentative de chaque image mono-composante, aptes à :

> calculer, pour chaque pixel de l'image mono-composante, une valeur dynamique locale sous forme d'une moyenne des valeurs des pixels compris dans un voisinage autour dudit pixel,
> calculer une moyenne des valeurs de dynamique locale de toute l'image mono-composante pour obtenir une valeur de dynamique globale de l'image mono-composante, la valeur de dynamique globale de l'image mono-composante formant ladite valeur représentative de chaque image mono-composante ;

des moyens de comparaison pour comparer (4424) les valeurs représentatives associées à chaque composante entre elles,
des moyens de détection pour détecter (4521) une présence d'une partie corporelle lorsqu'un écart entre la valeur représentative associée à la première image mono-composante et chaque valeur représentative associée à la deuxième image mono-composante est supérieur à un premier seuil prédéterminé.

**10.** Dispositif selon la revendication 8 ou 9, **caractérisé en ce que** la première composante est une composante rouge et chaque deuxième composante est une composante verte ou bleue.

**11.** Capteur d'empreintes digitales comprenant un dispositif selon l'une quelconque des revendications 6 à 10.

**12.** Programme d'ordinateur, comprenant des instructions pour mettre en oeuvre, par un dispositif, le procédé selon l'une quelconque des revendications 1 à 5, lorsque ledit programme est exécuté par une unité de calcul dudit dispositif.

**13.** Moyens de stockage, stockant un programme d'ordinateur comprenant des instructions pour mettre en oeuvre, par un dispositif, le procédé selon l'une quelconque des revendications 1 à 5, lorsque ledit programme est exécuté par une unité de calcul dudit dispositif.

**Patentansprüche**

**1.** Verfahren zur Erkennung einer Anwesenheit eines einen Fingerabdruck (D) tragenden Körperteils auf einem Fingerabdrucksensor (20), der eine transparente Seite (100C), auf die ein Körperteil (D) aufgelegt werden soll, einen gegenüber der transparenten Seite (100C) befindlichen Bildsensor (102), der fähig ist, ein Bild eines Fingerabdrucks zu erfassen, eine Lichtquelle (101A, 101B) enthält, die es ermöglicht, einen auf die transparente Seite (100C) aufgelegten Körperteil zu beleuchten, wobei das Verfahren enthält:

Einschalten (41) der Lichtquelle;
Erhalt (42) eines Mehrkomponentenbilds vom Bildsensor, wobei jede Farbkomponente des Mehrkomponentenbilds einem vordefinierten Wellenlängenbereich entspricht, wobei der Fingerabdrucksensor gemäß einem Totalreflexionsprinzip mit dunklem Hintergrund arbeitet, wobei die Lichtquelle ein Licht erzeugt, dessen Wellenlängen sich ausschließlich in einem der vordefinierten Wellenlängenbereiche befinden, Beleuchtungs-Wellenlängenbereich genannt;
Bilden (4301) eines Einkomponentenbilds ausgehend von jeder Komponente des Mehrkomponentenbilds,
Bestimmen (4302) eines für jedes Einkomponentenbild repräsentativen Werts, das enthält:

Berechnen, für jedes Pixel des Einkomponentenbilds, eines lokalen dynamischen Werts in Form eines Mittelwerts der Werte der in einer Nachbarschaft um das Pixel herum enthaltenen Pixel,
Berechnen eines Mittelwerts der Werte lokaler Dynamik des ganzen Einkomponentenbilds, um einen Wert globaler Dynamik des Einkomponentenbilds zu erhalten, wobei der Wert globaler Dynamik des Einkomponentenbilds den für jedes Einkomponentenbild repräsentativen Wert bildet;
Vergleich (4401, 4402) der für jede Komponente repräsentativen Werte miteinander,
Erkennen einer Anwesenheit (4501) eines Körperteils, wenn eine Abweichung zwischen dem für die Farbkomponente entsprechend dem Beleuchtungs-Wellenlängenbereich bestimmten repräsentativen Wert und den anderen repräsentativen Werten höher ist als eine erste vorbestimmte Schwelle.

**2.** Verfahren nach Anspruch 1, **dadurch gekennzeichnet, dass** jedes Pixel des Mehrkomponentenbilds eine rote Komponente, eine grüne Komponente und eine blaue Komponente enthält, wobei ein Verarbeitungsmodul ein Einkomponentenbild, das die rote Komponente enthält, ein Einkomponentenbild, das die grüne Komponente enthält und ein Einkomponentenbild bildet, das die blaue Komponente enthält.

**3.** Verfahren zur Erkennung einer Anwesenheit eines einen Fingerabdruck (D) tragenden Körperteils auf einem Fingerabdrucksensor (20), der eine transparente Seite (100C), auf die ein Körperteil (D) aufgelegt werden soll, einen gegenüber der transparenten Seite (100C) befindlichen Bildsensor (102), der fähig ist, ein Bild eines Fingerabdrucks zu erfassen, eine Lichtquelle (101A, 101B) enthält, die es ermöglicht, einen auf die transparente Seite (100C) aufgelegten Körperteil zu beleuchten, wobei das Verfahren enthält:

Einschalten (41) der Lichtquelle;
Erhalt (42) eines Mehrkomponentenbilds vom Bildsensor, wobei jede Farbkomponente des Mehrkomponentenbilds einem vordefinierten Wellenlängenbereich entspricht, wobei der Fingerabdrucksensor gemäß einem Totalreflexionsprinzip mit dunklem Hintergrund arbeitet, wobei die Lichtquelle ein Licht erzeugt, dessen Wellenlängen sich in einem vordefinierten ersten Wellenlängenbereich und in mindestens einem vordefinierten zweiten Wellenlängenbereich befinden,
Erhalt (4311) eines ersten Einkomponentenbilds vom Bildsensor, das die dem vordefinierten ersten Wellenlängenbereich entsprechende Komponente des Mehrkomponentenbilds enthält, und (4312) für jeden vordefinierten zweiten Wellenlängenbereich eines zweiten Einkomponentenbilds, das die dem vordefinierten zweiten Wellenlängenbereich entsprechende Komponente des Mehrkomponentenbilds enthält, wobei jedes Einkomponentenbild eine für ein vom Bildsensor für eine Farbkomponente generiertes Signal repräsentative Information ist;

Berechnen (4411) eines Differenzbilds zwischen dem ersten Bild und jedem zweiten Bild und zwischen jedem zweiten Bild;

Bestimmen (4412) eines für jedes Differenzbild repräsentativen Werts, das enthält:

Berechnen, für jedes Pixel des Differenzbilds, eines lokalen dynamischen Werts in Form eines Mittelwerts der Werte der Pixel, die in einer Nachbarschaft um das Pixel herum enthalten sind,

Berechnen eines Mittelwerts der Werte lokaler Dynamik des ganzen Differenzbilds, um einen Wert globaler Dynamik des Differenzbilds zu erhalten, wobei der Wert globaler Dynamik des Differenzbilds den für jedes Differenzbild repräsentativen Wert bildet;

Erkennen (4511) einer Anwesenheit eines Körperteils, wenn mindestens einer der repräsentativen Werte höher ist als eine erste vorbestimmte Schwelle.

4. Verfahren zur Erkennung einer Anwesenheit eines einen Fingerabdruck (D) tragenden Körperteils auf einem Fingerabdrucksensor (20), der eine transparente Seite (100C), auf die ein Körperteil (D) aufgelegt werden soll, einen gegenüber der transparenten Seite (100C) befindlichen Bildsensor (102), der fähig ist, ein Bild eines Fingerabdrucks zu erfassen, eine Lichtquelle (101A, 101B) enthält, die es ermöglicht, einen auf die transparente Seite (100C) aufgelegten Körperteil zu beleuchten, wobei das Verfahren enthält:

Einschalten (41) der Lichtquelle;

Erhalt (42) eines Mehrkomponentenbilds vom Bildsensor, wobei jede Farbkomponente des Mehrkomponentenbilds einem vordefinierten Wellenlängenbereich entspricht, wobei der Fingerabdrucksensor gemäß einem Totalreflexionsprinzip mit hellem Hintergrund arbeitet, wobei die Lichtquelle ein Licht erzeugt, dessen Wellenlängen sich in einem vordefinierten ersten Wellenlängenbereich und in mindestens einem vordefinierten zweiten Wellenlängenbereich befinden,

Erhalt eines ersten Einkomponentenbilds (4321) vom Bildsensor, das die dem vordefinierten ersten Wellenlängenbereich entsprechende Komponente des Mehrkomponentenbilds enthält, und für jeden vordefinierten zweiten Wellenlängenbereich eines zweiten Einkomponentenbilds (4322), das die dem vordefinierten zweiten Wellenlängenbereich entsprechende Komponente des Mehrkomponentenbilds enthält;

Berechnen (4423) eines für jedes Einkomponentenbild repräsentativen Werts, das enthält:

Berechnen, für jedes Pixel des Einkomponentenbilds, eines lokalen dynamischen Werts in Form eines Mittelwerts der Werte der Pixel, die in einer Nachbarschaft um das Pixel herum enthalten sind,

Berechnen eines Mittelwerts der Werte lokaler Dynamik des ganzen Einkomponentenbilds, um einen Wert globaler Dynamik des Einkomponentenbilds zu erhalten, wobei der Wert globaler Dynamik des Einkomponentenbilds den für jedes Einkomponentenbild repräsentativen Wert bildet;

Vergleich (4424) der jeder Komponente zugeordneten repräsentativen Werte miteinander,

Erkennen (4521) einer Anwesenheit eines Körperteils, wenn eine Abweichung zwischen dem dem ersten Einkomponentenbild zugeordneten repräsentativen Wert und jedem dem zweiten Einkomponentenbild zugeordneten repräsentativen Wert höher ist als eine erste vorbestimmte Schwelle.

5. Verfahren nach Anspruch 3 oder 4, **dadurch gekennzeichnet, dass** die erste Komponente eine rote Komponente und jede zweite Komponente eine grüne oder blaue Komponente ist.

6. Vorrichtung, die es ermöglicht, eine Anwesenheit eines einen Fingerabdruck (D) tragenden Körperteils auf einem Fingerabdrucksensor (20) zu erkennen, der eine transparente Seite (100C), auf die ein Körperteil (D) aufgelegt werden soll, einen gegenüber der transparenten Seite (100C) befindlichen Bildsensor (102), der fähig ist, ein Bild eines Fingerabdrucks zu erfassen, eine Lichtquelle (101A, 101B) enthält, die es ermöglicht, einen auf die transparente Seite (100C) aufgelegten Körperteil zu beleuchten, wobei die Vorrichtung enthält:

Einschalteinrichtungen (41), um die Lichtquelle einzuschalten;

Erhaltseinrichtungen (42), um ein Mehrkomponentenbild vom Bildsensor zu erhalten, wobei jede Komponente des Mehrkomponentenbilds einem vordefinierten Wellenlängenbereich entspricht, wobei der Fingerabdrucksensor fähig ist, gemäß einem Totalreflexionsprinzip mit dunklem Hintergrund zu arbeiten, wobei die Lichtquelle fähig ist, ein Licht zu erzeugen, dessen Wellenlängen sich ausschließlich in einem der vordefinierten Wellenlängenbereiche befinden, Beleuchtungs-Wellenlängenbereich genannt;

Bildungseinrichtungen, um ausgehend von jeder Komponente des Mehrkomponentenbilds ein Einkomponentenbild zu bilden (4301),

Bestimmungseinrichtungen, um einen für jedes Einkomponentenbild repräsentativen Wert zu bestimmen (4302), die fähig sind:

für jedes Pixel des Einkomponentenbilds einen lokalen dynamischen Wert in Form eines Mittelwerts der Werte der Pixel zu berechnen, die in einer Nachbarschaft um das Pixel herum enthalten sind,

einen Mittelwert der Werte lokaler Dynamik des ganzen Einkomponentenbilds zu berechnen, um einen Wert globaler Dynamik des Einkomponentenbilds zu erhalten, wobei der Wert globaler Dynamik des Einkomponentenbilds den repräsentativen Wert jedes Einkomponentenbilds bildet;

Vergleichseinrichtungen (4401, 4402), um die für jede Komponente repräsentativen Werte miteinander zu vergleichen,

Erkennungseinrichtungen, um eine Anwesenheit (4501) eines Körperteils zu erkennen, wenn eine Abweichung zwischen dem für die Farbkomponente entsprechend dem Beleuchtungs-Wellenlängenbereich bestimmten repräsentativen Wert und den anderen repräsentativen Werten höher ist als eine erste vorbestimmte Schwelle.

7. Vorrichtung nach Anspruch 6, **dadurch gekennzeichnet, dass** jedes Pixel des Mehrkomponentenbilds eine rote Komponente, eine grüne Komponente und eine blaue Komponente enthält, wobei die Vorrichtung ein Verarbeitungsmodul aufweist, das fähig ist, ein Einkomponentenbild, das die rote Komponente enthält, ein Einkomponentenbild, das die grüne Komponente enthält, und ein Einkomponentenbild zu bilden, das die blaue Komponente enthält.

8. Vorrichtung, die es ermöglicht, eine Anwesenheit eines einen Fingerabdruck (D) tragenden Körperteils auf einem Fingerabdrucksensor (20) zu erkennen, der eine transparente Seite (100C), auf die ein Körperteil (D) aufgelegt werden soll, einen gegenüber der transparenten Seite (100C) befindlichen Bildsensor (102), der fähig ist, ein Bild eines Fingerabdrucks zu erfassen, eine Lichtquelle (101A, 101B) enthält, die es ermöglicht, einen auf die transparente Seite (100C) aufgelegten Körperteil zu beleuchten, wobei die Vorrichtung enthält:

Einschalteinrichtungen (41) zum Einschalten der Lichtquelle;

Erhaltseinrichtungen (42), um ein Mehrkomponentenbild vom Bildsensor zu erhalten, wobei jede Komponente des Mehrkomponentenbilds einem vordefinierten Wellenlängenbereich entspricht, wobei der Fingerabdrucksensor fähig ist, gemäß einem Totalreflexionsprinzip mit dunklem Hintergrund zu arbeiten, wobei die Lichtquelle fähig ist, ein Licht zu erzeugen, dessen Wellenlängen sich in einem vordefinierten ersten Wellenlängenbereich und in mindestens einem vordefinierten zweiten Wellenlängenbereich befinden,

Erhaltseinrichtungen, um vom Bildsensor ein erstes Einkomponentenbild (4321), das die dem vordefinierten ersten Wellenlängenbereich entsprechende Komponente des Mehrkomponentenbilds enthält, und für jeden vordefinierten zweiten Wellenlängenbereich ein zweites Einkomponentenbild (4322) zu erhalten, das die dem vordefinierten zweiten Wellenlängenbereich entsprechende Komponente des Mehrkomponentenbilds enthält;

Recheneinrichtungen, um einen für jedes Einkomponentenbild repräsentativen Wert zu berechnen (4423), die fähig sind:

für jedes Pixel des Einkomponentenbilds einen lokalen dynamischen Wert in Form eines Mittelwerts der Werte der Pixel zu berechnen, die in einer Nachbarschaft um das Pixel herum enthalten sind,

einen Mittelwert der Werte lokaler Dynamik des ganzen Einkomponentenbilds zu berechnen, um einen Wert globaler Dynamik des Einkomponentenbilds zu erhalten, wobei der Wert globaler Dynamik des Einkomponentenbilds den für jedes Einkomponentenbilds repräsentativen Wert bildet;

Vergleichseinrichtungen, um die jeder Komponente zugeordneten repräsentativen Werte miteinander zu vergleichen (4424),

Erkennungseinrichtungen, um eine Anwesenheit eines Körperteils zu erkennen (4521), wenn eine Abweichung zwischen dem dem ersten Einkomponentenbild zugeordneten repräsentativen Wert und jedem dem zweiten Einkomponentenbild zugeordneten repräsentativen Wert höher ist als eine erste vorbestimmte Schwelle.

9. Vorrichtung, die es ermöglicht, eine Anwesenheit eines einen Fingerabdruck (D) tragenden Körperteils auf einem Fingerabdrucksensor (20) zu erkennen, der eine transparente Seite (100C), auf die ein Körperteil (D) aufgelegt werden soll, einen gegenüber der transparenten Seite (100C) befindlichen Bildsensor (102), der fähig ist, ein Bild eines Fingerabdrucks zu erfassen, eine Lichtquelle (101A, 101B) enthält, die es ermöglicht, einen auf die transparente Seite (100C) aufgelegten Körperteil zu beleuchten, wobei die Vorrichtung enthält:

Einschalteinrichtungen (41) zum Einschalten der Lichtquelle;

Erhaltseinrichtungen (42), um ein Mehrkomponentenbild vom Bildsensor zu erhalten, wobei jede Komponente des Mehrkomponentenbilds einem vordefinierten Wellenlängenbereich entspricht, wobei der Fingerabdrucksensor fähig ist, gemäß einem Prinzip der totalen Reflexion mit hellem Hintergrund zu arbeiten, wobei die Lichtquelle fähig ist, ein Licht zu erzeugen, dessen Wellenlängen sich in einem vordefinierten ersten Wellenlängenbereich und in mindestens einem vordefinierten zweiten Wellenlängenbereich befinden,

Erhaltseinrichtungen, um vom Bildsensor ein erstes Einkomponentenbild (4321), das die dem vordefinierten ersten Wellenlängenbereich entsprechende Komponente des Mehrkomponentenbilds enthält, und für jeden vordefinierten zweiten Wellenlängenbereich ein zweites Einkomponentenbild (4322) zu erhalten, das die dem vordefinierten zweiten Wellenlängenbereich entsprechende Komponente des Mehrkomponentenbilds enthält;

Recheneinrichtungen, um einen für jedes Einkomponentenbild repräsentativen Wert zu berechnen (4423), die fähig sind:

für jedes Pixel des Einkomponentenbilds einen lokalen dynamischen Wert in Form eines Mittelwerts der Werte der Pixel zu berechnen, die in einer Nachbarschaft und das Pixel herum enthalten sind,

einen Mittelwert der Werte lokaler Dynamik des ganzen Einkomponentenbilds zu berechnen, um einen Wert globaler Dynamik des Einkomponentenbilds zu erhalten, wobei der Wert globaler Dynamik des Einkomponentenbilds den für jedes Einkomponentenbild repräsentativen Wert bildet;

Vergleichseinrichtungen, um die jeder Komponente zugeordneten repräsentativen Werte miteinander zu vergleichen (4424),

Erkennungseinrichtungen, um eine Anwesenheit eines Körperteils zu erkennen (4521), wenn eine Abweichung zwischen dem dem ersten Einkomponentenbild zugeordneten repräsentativen Wert und jedem dem zweiten Einkomponentenbild zugeordneten repräsentativen Wert höher ist als eine erste vorbestimmte Schwelle.

10. Vorrichtung nach Anspruch 8 oder 9, **dadurch gekennzeichnet, dass** die erste Komponente eine rote Komponente und jede zweite Komponente eine grüne oder blaue Komponente ist.

11. Fingerabdrucksensor, der eine Vorrichtung nach einem der Ansprüche 6 bis 10 enthält.

12. Computerprogramm, das Anweisungen enthält, um das Verfahren nach einem der Ansprüche 1 bis 5 von einer Vorrichtung durchzuführen, wenn das Programm von einer Recheneinheit der Vorrichtung ausgeführt wird.

13. Speichereinrichtungen, die ein Computerprogramm speichern, das Anweisungen enthält, um das Verfahren nach einem der Ansprüche 1 bis 5 von einer Vorrichtung durchzuführen, wenn das Programm von einer Recheneinheit der Vorrichtung ausgeführt wird.

**Claims**

1. Method for detecting the presence of a body part bearing a fingerprint (D) on a fingerprint sensor (20) comprising a transparent face (100C) on which a body part (D) is to be placed, an image sensor (102), located opposite the transparent face (100C), able to acquire an image of a fingerprint, a light source (101A, 101B) for illuminating a body part placed on the transparent face (100C), the method comprising:

turning on (41) the light source;
obtaining (42) a multicomponent image from the image sensor, each colour component of the multicomponent image corresponding to a predefined wavelength range, the fingerprint sensor operating in accordance with a dark-background total internal reflection principle, the light source producing light the wavelengths of which are contained exclusively within one of the predefined wavelength ranges, referred to as illumination wavelength range;
forming (4301) a single-component image from each component of the multicomponent image, determining (4302) a representative value for each single-component image, comprising:

computing, for each pixel of the single-component image, a local dynamic value in the form of an average of the values of the pixels located within a vicinity around said pixel, computing an average of the local dynamic values of the entire single-component image so as to obtain an overall dynamic value for the single-component image, the overall dynamic value for the single-component image forming said representative value for each single-component image;

comparing (4401, 4402) the representative values for each component with one another, detecting the presence (4501) of a body part when a deviation between the representative value determined for the colour component corresponding to the illumination wavelength range and the other representative values is greater than a first predetermined threshold.

2. Method according to Claim 1, **characterized in that** each pixel of the multicomponent image comprises a red component, a green component and a blue component, a processing module forming a single-component image comprising the red component, a single-component image comprising the green component and a single-component image comprising the blue component.

3. Method for detecting the presence of a body part bearing a fingerprint (D) on a fingerprint sensor (20) comprising a transparent face (100C) on which a body part (D) is to be placed, an image sensor (102), located opposite the transparent face (100C), able to acquire an image of a fingerprint, a light source (101A, 101B) for illuminating a body part placed on the transparent face (100C), the method comprising:

turning on (41) the light source;
obtaining (42) a multicomponent image from the image sensor, each colour component of the multicomponent image corresponding to a predefined wavelength range, the fingerprint sensor operating in accordance with a dark-background total internal reflection principle, the light source producing light the wavelengths of which are located within a first predefined wavelength range and within at least one second predefined wavelength range,
obtaining (4311), from the image sensor, a first single-component image comprising the component of the multicomponent image corresponding to the first predefined wavelength range and, (4312) for each second predefined wavelength range, a second single-component image comprising the component of the multicomponent image corresponding to said second predefined wavelength range, each single-component image being information representative of a signal generated by said image sensor for a colour component;
computing (4411) a difference image between the first image and each second image and between each second image;

determining (4412) a representative value for each difference image, comprising:

computing, for each pixel of the difference image, a local dynamic value in the form of an average of the values of the pixels located within a vicinity around said pixel, computing an average of the local dynamic values of the entire difference image so as to obtain an overall dynamic value for the difference image, the overall dynamic value for the difference image forming said representative value for each difference image;

detecting (4511) the presence of a body part when at least one of the representative values is greater than a first predetermined threshold.

4. Method for detecting the presence of a body part bearing a fingerprint (D) on a fingerprint sensor (20) comprising a transparent face (100C) on which a body part (D) is to be placed, an image sensor (102), located opposite the transparent face (100C), able to acquire an image of a fingerprint, a light source (101A, 101B) for illuminating a body part placed on the transparent face (100C), the method comprising:

turning on (41) the light source;
obtaining (42) a multicomponent image from the image sensor, each colour component of the multicomponent image corresponding to a predefined wavelength range, the fingerprint sensor operating in accordance with a bright-background total internal reflection principle, the light source producing light the wavelengths of which are located within a first predefined wavelength range and within at least one second predefined wavelength range,
obtaining, from the image sensor, a first single-component image (4321) comprising the component of the multicomponent image corresponding to the first predefined wavelength range and, for each second predefined wavelength range, a second single-component image (4322) comprising the component of the multicomponent image corresponding to said second predefined wavelength range;
computing (4423) a representative value for each single-component image, comprising:

computing, for each pixel of the single-component image, a local dynamic value in the form of an average of the values of the pixels located within a vicinity around said pixel, computing an average of the local dynamic values of the entire single-component image so as to obtain an overall dynamic value for the single-component image, the overall dynamic value for the single-component image forming said representative value for each single-component image;

comparing (4424) the representative values associated with each component with one another, detecting (4521) the presence of a body part when a deviation between the representative value associated with the first single-component image and each representative value associated with the second single-component image is greater than a first predetermined threshold.

5. Method according to Claim 3 or 4, **characterized in that** the first component is a red component and each second component is a green or blue component.

6. Device for detecting the presence of a body part bearing a fingerprint (D) on a fingerprint sensor (20) comprising a transparent face (100C) on which a body part (D) is to be placed, an image sensor (102), located opposite the transparent face (100C), able to acquire an image of a fingerprint, a light source (101A, 101B) for illuminating a body part placed on the transparent face (100C), the device comprising:

turning-on means (41) for turning on the light source;
obtaining means (42) for obtaining a multicomponent image from the image sensor, each component of the multicomponent image corresponding to a predefined wavelength range, the fingerprint sensor being able to operate in accordance with a dark-background total internal reflection principle, the light source being able to produce light the wavelengths of which are contained exclusively within one of the predefined wavelength ranges, referred to as illumination wavelength range;
forming means for forming (4301) a single-component image from each component of the multicomponent image,
determining means for determining (4302) a representative value for each single-component image, able to:

compute, for each pixel of the single-component image, a local dynamic value in the form of an average of the values of the pixels located within a vicinity around said pixel,
compute an average of the local dynamic values of the entire single-component image so as to obtain an overall dynamic value for the single-component image, the overall dynamic value for the single-component image forming said representative value for each single-component image;

comparing means (4401, 4402) for comparing the representative values for each component with one another,
detecting means for detecting the presence (4501) of a body part when a deviation between the representative value determined for the colour component corresponding to the illumination wavelength range and the other representative values is greater than a first predetermined threshold.

7. Device according to Claim 6, **characterized in that** each pixel of the multicomponent image comprises a red component, a green component and a blue component, the device comprising a processing module able to form a single-component image comprising the red component, a single-component image comprising the green component and a single-component image comprising the blue component.

8. Device for detecting the presence of a body part bearing a fingerprint (D) on a fingerprint sensor (20) comprising a transparent face (100C) on which a body part (D) is to be placed, an image sensor (102), located opposite the transparent face (100C), able to acquire an image of a fingerprint, a light source (101A, 101B) for illuminating a body part placed on the transparent face (100C), the device comprising:

turning-on means (41) for turning on the light source;
obtaining means (42) for obtaining a multicomponent image from the image sensor, each component of the multicomponent image corresponding to a predefined wavelength range, the fingerprint sensor being able to operate in accordance with a dark-background total internal reflection principle, the light source being able to produce light the wavelengths of which are located within a first predefined wavelength range and within at least one second predefined wavelength range;
obtaining means for obtaining, from the image sensor, a first single-component image (4321) comprising the component of the multicomponent image corresponding to the first predefined wavelength range and, for each second predefined wavelength range, a second single-component image (4322) comprising the component of the multicomponent image corresponding to said second predefined wavelength range;
computing means for computing (4423) a representative value for each single-component image, able to:

compute, for each pixel of the single-component image, a local dynamic value in the form of an average of the values of the pixels located within a vicinity around said pixel, compute an average of the local dynamic values of the entire single-component image so as to obtain an overall dynamic value for the single-component image, the overall dynamic value for the single-component image forming said representative value for each single-component image;

comparing means for comparing (4424) the representative values associated with each component with one another,
detecting means for detecting (4521) the presence of a body part when a deviation between the representative value associated with the first single-component image and each representative value associated with the second single-component image is greater than a first predetermined threshold.

9. Device for detecting the presence of a body part bearing a fingerprint (D) on a fingerprint sensor (20) comprising a transparent face (100C) on which a body part (D) is to be placed, an image sensor (102), located opposite the transparent face (100C), able to acquire an image of a fingerprint, a light source (101A, 101B) for illuminating a body part placed on the transparent face (100C), the device comprising:

turning-on means (41) for turning on the light source;
obtaining means (42) for obtaining a multicomponent image from the image sensor, each component of the multicomponent image corresponding to a predefined wavelength range, the fingerprint sensor be able to operate in accordance with a bright-background total internal reflection principle, the light source being able to produce light the wavelengths of which are located within a first predefined wavelength range and within at least one second predefined wavelength range;
obtaining means for obtaining, from the image sensor, a first single-component image (4321) comprising the component of the multicomponent image corresponding to the first predefined wavelength range and, for each second predefined wavelength range, a second single-component image (4322) comprising the component of the multicomponent image corresponding to said second predefined wavelength range;
computing means for computing (4423) a representative value for each single-component image, able to:

compute, for each pixel of the single-component image, a local dynamic value in the form of an average of the values of the pixels located within a vicinity around said pixel, compute an average of the local dynamic values of the entire single-component image so as to obtain an overall dynamic value for the single-component image, the overall dynamic value for the single-component image forming said representative value for each single-component image;

comparing means for comparing (4424) the representative values associated with each component with one another,
detecting means for detecting (4521) the presence of a body part when a deviation between the representative value associated with the first single-component image and each representative value associated with the second single-component image is greater than a first predetermined threshold.

10. Device according to Claim 8 or 9, **characterized in that** the first component is a red component and each second component is a green or blue component.

11. Fingerprint sensor comprising a device according to any one of Claims 6 to 10.

12. Computer program, comprising instructions for a device to implement the method according to any one of Claims 1 to 5 when said program is executed by a computing unit of said device.

13. Storage means, storing a computer program comprising instructions for a device to implement the method according to any one of Claims 1 to 5 when said program is executed by a computing unit of said device.

D

100C  100

100A

$\alpha_A$  $\alpha_A$

100D

10

100B

101A

101B

102

Fig. 1

20

21

2

Fig. 2

211
CPU

212
RAM

213
ROM

21

210

HDD
214

COM
215

Fig. 3

41 — Allumer système d'éclairage

42 — Acquisition image multi-composantes

43 — Obtention informations représentatives

44 — Comparaison informations représentatives

45 — Détection présence

Fig. 4

Fig. 5A

Fig. 5B

Fig. 5C

**EP 3 471 016 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2915008 A1
- US 3200701 A
- FR 2757974